# EUROPEAN PATENT APPLICATION

(11) **EP 3 238 714 A1**
(43) Date of publication of application: **01.11.2017**
(21) Application number: 17168465.7
(22) Date of filing: 27.04.2017
(51) Int. Cl.: A61K 9/20, A61K 9/24, A61K 31/135, A61K 31/192

(54) **EXTENDED RELEASE FORMULATIONS OF FLURBIPROFEN AND TRAMADOL**

(30) Priority: 28.04.2016 TR 201605504; 02.05.2016 TR 201605632
(71) Applicant: Sanovel Ilac Sanayi ve Ticaret A.S., 34460 Istanbul (TR)
(72) Inventor: TÜRKYILMAZ, Ali, 34460 Istanbul (TR); DURGUN, Deniz Ömür, 34460 Istanbul (TR); PEHLIVAN AKALIN, Nur, 34460 Istanbul (TR); HATIRNAZ, Zekiye Basak, 34460 Istanbul (TR)
(74) Representative: Sevinç, Erkan

(57) **Abstract**

The present invention relates to a pharmaceutical formulation comprising an immediate release phase comprising an effective amount of flurbiprofen and a controlled release phase comprising an effective amount of tramadol and pharmaceutically acceptable excipients.

## Description

### Field of the Invention

The present invention relates to a pharmaceutical formulation comprising an immediate release phase comprising an effective amount of flurbiprofen or a pharmaceutically acceptable salt thereof and a controlled release phase comprising an effective amount of tramadol or a pharmaceutically acceptable salt thereof, and pharmaceutically acceptable excipients.

### Background of the Invention

Flurbiprofen is a propionic acid derivative which is an NSAID (non-steroidal anti-inflammatory drug), having analgesic and anti-inflammatory activities. Its chemical structure is illustrated with Formula 1 given below.

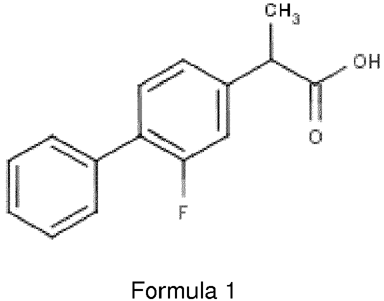

Flurbiprofen is used for alleviating pain in muscle-skeleton system and joint disorders such as ankylosing spondylitis, osteoarthritis, and rheumatoid arthritis, in soft tissue injuries such as sprains and strains, in postoperative cases, and in painful severe menstruation and migraine. It is in the market under the brandname of ANSAID^{®} in strength of 50, 100, 200 and 300 mg. It is recommended 2, 3 or 4 times a day dose.

Another molecule that is used as an analgesic is tramadol disclosed in the patent US6339105 (B1). Tramadol is a centrally acting synthetic opioid analgesic. The chemical name for tramadol hydrochloride is (±)cis-2-[(dimethylamino)methyl]-1-(3methoxyphenyl) cyclohexanol hydrochloride. Although its mode of action is not completely understood, from animal tests, at least two complementary mechanisms appear applicable: binding of parent and M1 metabolite to µ-opioid receptors and weak inhibition of re-uptake of norepinephrine and serotonin.

Use of flurbiprofen and tramadol in combination may cause some, particularly gastrointestinal, side effects. Flurbiprofen shows burning sensation in the gastrointestinal system and tramadol shows nausea, vomiting, dizziness, dry mouth, and sedation. Moreover, the use of tramadol with flurbiprofen can cause additional effects. Avoiding these systemic adverse effects of flurbiprofen and tramadol is very important for the patient.

Instead of one per se use, combining more than one molecule in one dosage form increases the patients' quality of life and patients' compliance. In literature, the combination of flurbiprofen and tramadol has been studied. However, there is no study on controlled release combination of flurbiprofen and tramadol in a same dosage form.

Controlled-release dosage forms are designed to release a drug at a predetermined rate by maintaining a constant drug level for a specific period of time with minimum side effects. Compared to immediate release formulations, a controlled release formulation containing a physiologically active drug allows blood concentrations of the drug to be maintained for a long time or above the therapeutic concentration. By providing a controlled-release formulation of tramadol, it may be possible to reduce the frequency of administration, while providing the same or better therapeutic effects, potentially improving compliance. The controlled-release formulation may avoid a rapid increase in blood plasma concentration levels immediately after administration of the drug, thus potentially reducing or eliminating adverse side effects.

Considering all these, controlled release combinations of flurbiprofen and tramadol in a suitable pharmaceutical dosage formulation is needed. In this present invention, a formulation has been developed as to combine an immediate release phase for flurbiprofen and a controlled release phase for tramadol.

### Description of the Invention

The present invention relates to a pharmaceutical composition comprising an immediate release phase comprising an effective amount of flurbiprofen or a pharmaceutically acceptable salt thereof and a controlled release phase comprising an effective amount of tramadol or a pharmaceutically acceptable salt thereof, and pharmaceutically acceptable excipients.

According to one embodiment, the pharmaceutical acceptable salt of tramadol is tramadol HCl.

In this embodiment of this present invention, a fixed dose combination comprising comprising an immediate release phase comprising flurbiprofen and a controlled release phase comprising tramadol HCl has been developed for the management of moderately severe pain in adults. An improved analgesic effect has been achieved with the synergistic effect of flurbiprofen and controlled release tramadol.

In one embodiment, in this present invention, pharmaceutical combination formulated in one dosage form providing immediate release for flurbiprofen and controlled release for tramadol HCl.

Using different drugs may induce incompatibility problems and undesired dissolution profiles that cause undesired side effects. In this present invention, a pharmaceutical dosage form comprising flurbiprofen in combination with controlled release tramadol has been developed with safe and effective dissolution profiles for each drug molecule.

The term "controlled release" means that prolonged release or sustained release or modified release or extended release or delayed release or retarded release or gradual release or programmed release. The phase has a rate controlling agent that allows it to dissolve slowly either in the stomach or small intestine. The active ingredient is then slowly dissolved, therefore slowly absorbed into the bloodstream. So the rate controlling agents affect dissolution rate or dissolution profile of the active ingredient.

An embodiment of this present invention is to provide a pharmaceutical formulation making the plasma concentration level stable by maintaining release of tramadol in the blood stream for a longer time period sufficient to justify once daily or twice daily dosing and thus increases patient compliance.

According to one embodiment, flurbiprofen is present in an amount of between 10mg and 300mg, preferably between 10mg and 200mg and more preferably it is in an amount of between 50mg and 200mg.

According to one embodiment, tramadol HCl is present in an amount of between 10mg and 400mg, preferably between 20mg and 300mg and more preferably it is in an amount of between 35mg and 150mg.

According to one embodiment, the ratio of flurbiprofen to tramadol HCl is in the range of 0.01 to 30 (w/w), preferably 0.025 to 15 (w/w), more preferably 0.025 to 3 (w/w).

According to one embodiment, the ratios used in this present invention ensure the required effective doses for the treatment and immediate release for flurbiprofen and desired controlled release for tramadol HCl.

An embodiment of this present invention is to combine flurbiprofen and tramadol HCl in a same and stable dosage form with desired dissolution profiles. Pharmaceutical formulation of this present invention is in the form of tablet, bilayer tablet, trilayer tablet, multilayer tablet, capsule, tablet in tablet, an inlay tablet, injectable preparate, suspension, syrup, sachet, ointment, cream or gel.

In one embodiment, pharmaceutical formulation of this present invention is in the form of a tablet or a bilayer tablet or a trilayer tablet.

In one embodiment, pharmaceutical formulation of this present invention is in the form of a tablet.

In one embodiment, pharmaceutical formulation of this present invention is in the form of a bilayer tablet.

In one embodiment, pharmaceutical formulation of this present invention is in the form of a trilayer tablet.

According to this embodiment, the pharmaceutical formulation of this present invention comprises at least one pharmaceutically acceptable excipient.

According to this embodiment, at least one pharmaceutically acceptable excipient is selected from a group comprising binders, disintegrants, diluents, lubricants and glidants or mixtures thereof.

In one embodiment binder is selected from the group comprising polyethylene oxide, microcrystalline cellulose (PH 101, PH 102), polyvinylpyrrolidone, crospovidon, sugars, glycose syrups, natural gums, guar gum, gelatins, pullulan, agar, alginate, sodium alginates, K-Carrageenan, glycyrrhizin, polymetacrylates, collagen, agar, , hyaluronic acid, pectin, tragachanti gum, carboxymethyl cellulose, polyethylene glycol, polyvinyl alcohol, polyvinyl acetate and their copolymers, cellulose derivatives such as hydroxypropyl methyl cellulose, carboxy methyl cellulose, methyl cellulose, polyvinylalcohol, carrageenan, carbomer, poloxamer, polyacrylamide, aluminum hydroxide, benthonite, laponite, setostearyl alcohol, polyoxyethylene-alkyl ethers, acacia mucilage, polydextrose, xylitol, sucrose stearate or mixtures thereof.

In this embodiment, in order to achieve controlled release for tramadol HCl, polyethylene oxide has been used as a binder. During the development study to combine flurbiprofen and tramadol HCl, by using polyethylene oxide desired controlled release for tramadol HCl has been achieved. Moreover, immediate release profile of flurbiprofen has remain intact.

With the synergistic effect of polyethylene oxide used in the formulation, desired dissolution has been achieved for both flurbiprofen and tramadol HCl.

According to this embodiment, in this formulation of this present invention, binder is polyethylene oxide. The amount of polyethylene oxide is between 1.00 - 40.00 %, preferably 5.00 -40.0 % and more preferably it is 5.00-30.00 % by weight of total formulation.

Another embodiment of this invention, the pharmaceutical composition comprising flurbiprofen and tramadol is administrated once a day (QD) or twice a day (BID) and preferably once a day.

Suitable disintegrants are selected from the group selected from the group comprising polyethylene oxide, sodium starch glycolate, microcrystalline cellulose, croscarmellose sodium, sodium carboxymethyl starch, soy polysaccharide, cross-linked alginic acid, crospovidone, copovidone, gellan gum, xanthan gum, calcium silicate or ion exchange resins or mixtures thereof.

Suitable diluents are selected from the group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

Suitable lubricants are selected from sodium stearyl fumarate, magnesium stearate, polyethylene glycol, sodium lauryl sulphate, magnesium lauryl sulphate, fumaric acid, glyceryl palmitostearate, hydrogenated natural oils, zinc stearate, calcium stearate, silica, talc, stearic acid, polyethylene glycol, paraffin or mixtures thereof.

Suitable glidants are selected from colloidal silicon dioxide, aluminium silicate or mixtures thereof.

According to one embodiment, the pharmaceutical composition is in the form of a tablet comprising;
a) 5.0 - 80.0% flurbiprofen,
b) 5.0 - 80.0% tramadol HCl,
c) 0.1 - 90.0% lactose,
d) 8.0 - 45 % microcrystalline cellulose,
e) 0.5 - 20.0% glyceryl behanate,
f) 1.0 - 15.0% ammonium methacrylate copolymer (Eudragit RS 30D),
g) 1.0 - 40.0 % polyethylene oxide,
h) 0.5 - 5.0% colloidal silicon dioxide,
i) 0.1 - 10.0% magnesium stearate by weight of total formulation.

According to one embodiment, the pharmaceutical composition is in the form of a bilayer tablet comprising;
- flurbiprofen layer:
   a) 5.0 - 80.0% flurbiprofen,
   b) 0.1 - 90.0% lactose,
   c) 8.0 - 45.0 % microcrystalline cellulose,
   d) 0.5 - 20.0% glyceryl behanate,
   e) 1.0 - 15.0% ammonium methacrylate copolymer (Eudragit RS 30D),
   f) 0.3 - 5.0% colloidal silicon dioxide,
   g) 0.1 - 10.0% magnesium stearate
- tramadol HCl layer:
   h) 5.0 - 80.0 % tramadol HCl,
   i) 1.0 - 40.0 % polyethylene oxide,
   j) 0.1 - 90.0 % polyvinyl prolidone,
   k) 1.0 - 40.0 % calcium hydrogen phosphate dihydrate,
   l) 0.1 - 5.0% colloidal silicon dioxide,
   m) 0.1 - 10.0% magnesium stearate by weight of total formulation.

### Example 1: Tablet

| **ingredient** | **amount %** |
|---|---|
| Flurbiprofen | 33.3 % |
| Tramadol HCl | 16.7 % |
| Lactose monohydrate | 9.0 % |
| Microcrystalline cellulose | 8.0 % |
| Polyethylene oxide | 17.0 % |
| Glyceryl behanate | 8.4 % |
| Ammonium methacrylate copolymer | 5.4 % |
| Colloidal silicon dioxide | 0.5 % |
| Magnesium stearate | 0.35 % |

The process of the formulations is carried out as follows:
Flurbiprofen, Tramadol HCl, lactose monohydrate, microcrystalline cellulose, glyceryl behanate are mixed. Powder mixture is granulated with Eudragit RS 30 D. This mixture is sieved and dried in flued bed dryer. Then, sieved again. Colloidal silicon dioxide is added to this mixture. Then, magnesium stearate is added to this mixture. Powder mixture is pressed into tablets.

### Example 2: Bilayer Tablet

| **ingredient** | **amount %** |
|---|---|
| **Flurbiprofen layer** | |
| Flurbiprofen | 27.7 % |
| Lactose monohydrate | 11.4 % |
| Microcrystalline cellulose | 8.3 % |
| Glyceryl behanate | 4.72 % |
| Ammonium methacrylate copolymer | 3 % |
| Colloidal silicon dioxide | 0.3 % |
| Magnesium stearate | 0.2 % |

| **Tramadol layer** | |
|---|---|
| Tramadol HCl | 14.0 % |
| Polyethylene oxide | 20.7 % |
| Polyvinyl prolidone, | 0.76 % |
| Calcium hydrogen phosphate dihydrate | 8.41 % |
| Colloidal silicon dioxide | 0.23 % |
| Magnesium stearate | 0.4 % |

The process of the formulations is carried out as follows:

### Flurbiprofen layer:

Flurbiprofen, lactose (SuperTab 11 SD), microcrystalline cellulose and glyceryl behanate are mixed and sieved. Powder mixture is granulated with ammonium methacrylate copolymer (Eudragit RS 30 D). Granules are sieved and dried in flued bed dryer. Colloidal silicon dioxide is added and mixed. Then, magnesium stearate is added and mixed.

### Tramadol layer:

Tramadol HCl, polyvinyl prolidone (K-90), a half of polyethylene oxide and calcium hydrogen phosphate dihydrate are mixed. The mixture is granulated in compactor and sieved. Other part of polyethylene oxide and colloidal silicon dioxide are added to the granules and mixed. Then, magnesium stearate is added and mixed again.

These two mixtures for each layer are pressed into bilayer tablets.

### Example 3: Tramadol XR Tablet

| **ingredient** | **amount %** |
|---|---|
| Tramadol HCl | 31.0 % |
| Polyethylene oxide | 46.4 % |
| Polyvinyl prolidone, | 1.7 % |
| Calcium hydrogen phosphate dihydrate | 19.0 % |
| Colloidal silicon dioxide | 0.5 % |
| Magnesium stearate | 1.1 % |

The process of the formulations is carried out as follows: Tramadol HCl, polyvinyl prolidone (K-90), a half of polyethylene oxide and calcium hydrogen phosphate dihydrate are mixed. The mixture is granulated in compactor and sieved. Other part of polyethylene oxide and colloidal silicon dioxide are added to the granules and mixed. Then, magnesium stearate is added and mixed again.

## Claims

1. A pharmaceutical formulation comprising an immediate release phase comprising an effective amount of flurbiprofen or a pharmaceutically acceptable salt thereof and a controlled release phase comprising an effective amount of tramadol or a pharmaceutically acceptable salt thereof, and pharmaceutically acceptable excipients.

2. The pharmaceutical formulation according to claim 1, wherein the pharmaceutical acceptable salt of tramadol is tramadol HCl.

3. The pharmaceutical formulation according to claim 1, flurbiprofen is present in an amount of between 10mg and 300mg, preferably between 10mg and 200mg and more preferably it is in an amount of between 50mg and 200mg.

4. The pharmaceutical formulation according to claim 2, tramadol HCl is present in an amount of between 10mg and 400mg, preferably between 20mg and 300mg and more preferably it is in an amount of between 35mg and 150mg.

5. The pharmaceutical formulation according to claim 1 or 2, wherein the ratio of flurbiprofen to tramadol HCl is in the range of 0.01 to 30 (w/w), preferably 0.025 to 15 (w/w), more preferably 0.025 to 3 (w/w).

6. The pharmaceutical formulation according to claim 1, wherein said pharmaceutical formulation is in the form of tablet, bilayer tablet, trilayer tablet, multilayer tablet, capsule, tablet in tablet, an inlay tablet, injectable preparate, suspension, syrup, sachet, ointment, cream or gel.

7. The pharmaceutical formulation according to claim 6, wherein said pharmaceutical composition is in the form of a tablet or a bilayer tablet or a trilayer tablet.

8. The pharmaceutical formulation according to claim 1, wherein at least one pharmaceutically acceptable excipient is selected from a group comprising binders, disintegrants, diluents, lubricants and glidants or mixtures thereof.

9. The pharmaceutical formulation according to claim 8, wherein binder is polyethylene oxide.

10. The pharmaceutical formulation according to claim 9, wherein the amount of polyethylene oxide is between 1.00 - 40.00 %, preferably 5.00 -40.0 % and more preferably it is 5.00-30.00 % by weight of total formulation.

11. The pharmaceutical formulation according to claim 7, in the form of a tablet comprising;
a) 5.0 - 80.0% flurbiprofen,
b) 5.0 - 80.0% tramadol HCl,
c) 0.1 - 90.0% lactose,
d) 8.0 - 45 % microcrystalline cellulose,
e) 0.5 - 20.0% glyceryl behanate,
f) 1.0 - 15.0% ammonium methacrylate copolymer,
g) 1.0 - 40.0 % polyethylene oxide,
h) 0.5 - 5.0% colloidal silicon dioxide,
i) 0.1 - 10.0% magnesium stearate by weight of total formulation.

12. The pharmaceutical formulation according to claim 7, in the form of a bilayer tablet comprising;
• flurbiprofen layer:
a) 5.0 - 80.0% flurbiprofen,
b) 0.1 - 90.0 % lactose,
c) 8.0 - 45.0 % microcrystalline cellulose,
d) 0.5 - 20.0% glyceryl behanate,
e) 1.0 - 15.0% ammonium methacrylate copolymer,
f) 0.3 - 5.0% colloidal silicon dioxide,
g) 0.1 - 10.0% magnesium stearate
• tramadol HCl layer:
h) 5.0 - 80.0 % tramadol HCl,
i) 1.0 - 40.0 % polyethylene oxide,
j) 0.1 - 90.0 % polyvinyl prolidone,
k) 1.0 - 40.0 % calcium hydrogen phosphate dihydrate,
l) 0.1 - 5.0% colloidal silicon dioxide,
m) 0.1 - 10.0% magnesium stearate by weight of total formulation.
